# EUROPÄISCHE PATENTANMELDUNG

(11) **EP 2 963 451 A1**
(43) Veröffentlichungstag der Anmeldung: **06.01.2016**
(21) Anmeldenummer: 15174475.2
(22) Anmeldetag: 30.06.2015
(51) Int. Cl.: G01T 1/02

(54) **DOSISMESSGERÄT ZUR MESSUNG DER AUGENLINSENDOSIS**

(30) Priorität: 01.07.2014 DE 202014005506 U
(71) Anmelder: Berthold Technologies GmbH & Co. KG, 75323 Bad Wildbad (DE); Karlsruher Institut für Technologie, 76131 Karlsruhe (DE)
(72) Erfinder: Klett, Dr. Alfred, 75305 Neuenbürg (DE); Becker, Dr. Frank, 76297 Stutensee (DE)
(74) Vertreter: Patentanwälte Ruff, Wilhelm, Beier, Dauster & Partner mbB

(57) **Zusammenfassung**

Ein Dosismessgerät zur Messung der Augenlinsendosis umfasst eine für eintretende ionisierende Strahlung teilweise absorbierende Absorberschicht (120), die an einer Strahlungseintrittsseite (110) des Dosismessgeräts (100) angeordnet ist und eine zwischen einer Eintrittsfläche und einer Austrittsfläche der Absorberschicht gemessene effektive Absorberschichtdicke (D1) aufweist, eine auf die Austrittsfläche der Absorberschicht in Durchstrahlungsrichtung folgende Detektorschicht (130), die eine zwischen einer Eintrittsfläche und einer Austrittsfläche der Detektorschicht gemessene wirksame Detektorschichtdicke (D2) aufweist, und eine der Detektorschicht zugeordnete Auswerteeinrichtung (190) zur Bestimmung der in der Detektorschicht deponierten Strahlungsenergie der ionisierenden Strahlung. Die effektive Absorberschichtdicke (D1) ist an ein Absorberschichtmaterial oder eine Kombination mehrerer Absorberschichtmaterialien so angepasst, dass die Absorberschicht in Bezug auf die Wirkung auf hindurchtretende ionisierende Strahlung einer Schicht aus gewebeäquivalentem Material mit einer Schichtdicke zwischen 2 mm und 4 mm entspricht. Die Detektorschichtdicke (D2) liegt bei einem Festkörperdetektor oder Flüssigkeitsdetektor im Bereich von 0.01 mm bis 2 mm und bei einem gasgefüllten Detektor im Bereich von 1 mm bis 100 mm. Die Auswerteeinrichtung ist zur unmittelbaren Bestimmung der in der Detektorschicht deponierten Strahlungsenergie der ionisierenden Strahlung konfiguriert.

## Beschreibung

Die Erfindung betrifft ein Dosismessgerät zur Messung der Augenlinsendosis. Ein Dosismessgerät ist ein Messgerät, welches z.B. im Strahlenschutz zur Messung der Dosis verwendet werden kann, um die Effekte ionisierender Strahlung auf den Menschen zu quantifizieren.

Es gibt in der Metrologie verschiedene Dosisdefinitionen. Daher sollten Dosismessgeräte speziell für spezifische Messaufgaben ausgelegt werden. Beispielsweise gibt es Dosismessgeräte zur Messung der Hautdosis und Dosismessgeräte zur Messung der Augenlinsendosis.

Die EP 2 469 304 A2 beschreibt ein Dosismessgerät mit einem Detektor zur Messung der Richtungs-Äquivalentdosis H'(0,07, Ω), die zur Abschätzung einer Hautdosis herangezogen werden kann.

Für die Messung der Augenlinsendosis gibt es passive Augenlinsendosimeter auf Thermolumineszenzbasis (sogenannte TLD Dosismessgeräte). Ein solches Augenlinsen-Dosismessgerät ist unter der Bezeichnung EYE-D™ der Firma Radcard s.c., Krakau, Polen erhältlich.

Die GB 2 227 633 A beschreibt ein passives Dosismessgerät, das in der Lage ist, sowohl die Hautdosis als auch die Augenlinsendosis zu messen. Das Dosismessgerät hat unterschiedliche Phosphor-Elemente, wobei eines zur Messung der Augenlinsendosis und eines zur Messung der Hautdosis vorgesehen ist. Die Phosphor-Elemente sind in Ausnehmungen auf gegenüberliegenden Seiten einer Platte in der Weise untergebracht, dass das Phosphor-Element zur Messung der Hautdosis in Einstrahlungsrichtung der zu messenden Strahlung über dem Phosphor-Element zur Messung der Augenlinsendosis liegt, so dass es auch als Filter für diejenige Strahlung dient, die zum Phosphor für die Augenlinsendosismessung hindurchritt.

Bei passiven Dosismessgeräten stehen die Daten nicht unmittelbar, sondern erst mit zeitlicher Verzögerung zur Verfügung, üblicherweise im Zeitraum von mehreren Tagen bis über einen Monat. Durch die integrierende Wirkung derartiger Dosismessgeräte ist nicht festzustellen, an welchen Orten und zu welchen Zeiten die Strahlenbelastung besonders stark war.

Die Trübung der Augenlinse (Katarakt) infolge Bestrahlung mit ionisierender Strahlung ist ein Effekt, der dazu geführt hat, im Rahmen der Strahlenschutzvorsorge die Exposition der Augenlinse speziell zu begrenzen. Die International Commission on Radiological Protection (ICRP) hat zum Ausschluss von deterministischen Schäden (im Wesentlichen die Trübung der Augenlinse) spezielle Dosisgrenzwerte für die Augenlinse festgelegt. Die Dosisgrenzwerte lagen bei 150 mSv pro Jahr für beruflich strahlenexponierte Erwachsene und bei 15 mSv pro Jahr für allen anderen Personen (ICRP 1991). Lange Zeit wurde die Kataraktbildung als Folge einer Exposition mit ionisierender Strahlung als deterministischer Effekt angesehen, bei dem eine Schwellendosis (> 500 mSv) existiert, unterhalb der kein nachweisbarer Effekt auftritt.

Diese Ansicht ist in den letzten Jahren aufgrund neuerer Untersuchungen vermehrt bezweifelt worden. Es wird gefordert, die Schwellendosis wesentlich niedriger anzusetzen, wobei nicht klar ist, ob überhaupt eine solche existiert.

Es wird somit ein Bedarf gesehen, im Sinne eines optimierten Strahlenschutzes die Augenlinsendosis mit relativ einfachen Anordnungen direkt präzise messen zu können. Als "direkte Messung" wird hier eine Messung bezeichnet, die in der Lage ist, eine für einen bestimmten Messort geltende Dosis zu messen und die Messergebnisse praktisch zeitgleich mit der Messung oder nur mit geringer zeitlicher Verzögerung zur Verfügung zu stellen.

### AUFGABE UND LÖSUNG

Es ist eine Aufgabe der Erfindung, ein zuverlässiges und in der Handhabung einfaches Dosismessgerät bereitzustellen, das speziell an eine möglichst exakte Messung der Augenlinsendosis angepasst und hierfür besonders geeignet ist. Das Dosismessgerät sollte eine spezifisch für die Augenlinsendosismessung dosisrichtige Messung ionisierender Strahlung mit guter örtlicher und zeitlicher Auflösung erlauben.

Diese Aufgabe wird gelöst durch ein Dosismessgerät mit den Merkmalen von Anspruch 1. Vorteilhafte Weiterbildungen sind in den abhängigen Ansprüchen angegeben. Der Wortlaut sämtlicher Ansprüche wird durch Bezugnahme zum Inhalt der Beschreibung gemacht.

Für die Messung der Dosis der Augenlinse werden die für den Strahlenschutz definierten Dosismessgrößen verwendet, um durch eine Messung einen Schätzwert für die applizierte Augenlinsendosis zu erhalten. Die International Commission on Radiation Units & Measurements (ICRU) hat speziell für die Überwachung der Augenlinse die Ortsdosis-Messgröße H'(3,Ω) und die Personendosis-Messgröße Hp(3) definiert, die sich auf die Äquivalentdosis in 3 mm Tiefe in der ICRU-Kugel bzw. im Gewebe beziehen (ICRU Report 85: Fundamental Quantities and Units for Ionizing Radiation). Die Tiefe von 3 mm orientiert sich daran, dass die Augenlinse hinter der Hornhaut und vor der vorderen Augenkammer in ca. 2 mm bis 4 mm Tiefe im Auge liegt. Diese Messgrößen sind über die sogenannte ICRU Kugel (Phantom zur Nachbildung des Menschen hinsichtlich der Energieaufnahme durch ionisierende Strahlung in Form einer Kugel mit 30 cm Durchmesser mit gewebeäquivalenter Materialzusammensetzung) als Äquivalentdosis in einer Tiefe von 3 mm unter der Kugeloberfläche definiert. Sie umfasst die Wirkung der Strahlung, die in diese Tiefe eindringen kann, und beinhaltet auch deren Rückstreuung am inneren Kugelkörper. H'(3,Ω) und H_{P}(3) wurden jedoch nicht mit der Novelle von Strahlenschutz- und Röntgenverordnung in den Jahren 2001 und 2002 eingeführt. Die damals neuen gesetzlichen Dosis-Messgrößen sind für die Personendosimetrie die Tiefen-Personendosis H_{P}(10) für Ganzkörpermessungen (zur Abschätzung der effektiven Dosis) in 10 mm Tiefe in der ICRU-Kugel und die Oberflächen-Personendosis H_{P}(0,07) für Teilkörperdosismessungen (zur Abschätzung der lokalen Hautdosis) in 0,07 mm Tiefe. Als Messgrößen für die Ortsdosis sind analog die Umgebungs-Äquivalentdosis H*(10) und Richtungs-Äquivalentdosis H'(0,07,Ω) gültig.

Ein Vorteil der beanspruchten Erfindung besteht darin, dass das Dosismessgerät so ausgelegt ist, dass bedingt durch die Konstruktion des Dosismessgeräts bzw. seiner Komponenten die Definition dieser Messgröße (Augenlinsendosis) möglichst genau messtechnisch nachgebildet wird. Dadurch ist eine dosisrichtige Messung möglich. Aufgrund der unmittelbaren Auswertung ist eine aktive Messung möglich, um lokal auftretende Dosen ortstaufgelöst bestimmen und zeitnah oder simultan zur Messung quantitativ bewerten zu können. Insbesondere ist eine Messung der Ortsdosismessgröße H'(3,Ω) möglich.

Das Dosismessgerät umfasst an seiner Strahlungseintrittsseite eine für eintretende ionisierende Strahlung teilweise absorbierende Absorberschicht, deren effektive Absorberschichtdicke zwischen einer Eintrittsfläche und einer Austrittsfläche der Absorberschicht gemessen wird. Um diese insgesamt angestrebte Absorptionswirkung zu erreichen, kann die Absorberschicht aus einem Absorberschichtmaterial oder aus einer Kombination mehrerer Absorberschichtmaterialien bestehen, wobei das Material bzw. die Materialien in ihrer Gesamtheit so an die Messaufgabe angepasst sind, dass die Absorberschicht die gewünschte Absorptionswirkung hat. Die Absorberschicht kann z.B. durch eine einzige Schicht aus einem Absorberschichtmaterial gebildet sein. Es ist jedoch auch möglich, dass die Absorberschicht aus mehreren Einzelschichten bzw. Teilschichten zusammengesetzt ist, welche entweder in direktem flächigen Kontakt miteinander stehen oder aber einen gegenseitigen Abstand aufweisen können. Entscheidend ist hierbei die insgesamt auf die hindurchtretende ionisierende Strahlung wirkende Absorptionswirkung zwischen Eintrittsfläche und Austrittsfläche. Die Absorberschicht ist so ausgelegt, dass sie in Bezug auf die Wirkung auf hindurchtretende ionisierende Strahlung einer Schicht aus gewebeäquivalentem Material mit einer Schichtdicke zwischen 2 mm und 4 mm entspricht. Die Absorberschicht kann als Eintrittsfenster des Dosismessgeräts angesehen werden.

Die effektive Absorberschichtdicke ist vorzugsweise so bemessen, dass ihre Absorptionswirkung einer Schicht aus gewebeäquivalentem Material mit einer Schichtdicke zwischen 2,5 mm und 3,5 mm, insbesondere zwischen 2,8 mm und 3,2 mm, entspricht, z.B. einer Schichtdicke von etwa 3 mm.

Auf die Austrittsfläche der Absorberschicht folgt in Durchstrahlungsrichtung eine Detektorschicht. Die Detektorschicht kann aus einem für eintretende ionisierende Strahlung empfindlichen festen Detektorschichtmaterial (ein Material oder eine Mischung oder Kombination mehrerer Materialien) bestehen, so dass ein Festkörperdetektor gebildet wird. Es ist auch möglich, dass die Detektorschicht aus einem für eintretende ionisierende Strahlung empfindlichen flüssigen Detektorschichtmaterial (eine Flüssigkeit oder eine Mischung oder Kombination mehrerer Flüssigkeiten) besteht (Flüssigkeitsdetektor). Es ist auch möglich, dass die Detektorschicht durch eine Anordnung mehrerer aufeinander abgestimmter Komponenten besteht, die einen gasgefüllten Detektor bilden und so zusammenwirken, dass mittels der Komponenten bzw. des gasgefüllten Detektors ionisierende Strahlung quantitativ detektiert werden kann.

Auf die Detektorschicht trifft im Wesentlichen derjenige Anteil der ionisierenden Strahlung auf, der nicht durch die Absorberschicht absorbiert wurde. Die Detektorschicht hat eine zwischen einer Eintrittsfläche und einer Austrittsfläche gemessene wirksame Detektorschichtdicke. In der Detektorschicht wird die Strahlungsenergie der durch die Absorberschicht nicht absorbierten eintretenden ionisierenden Strahlung auf eine durch die Art der Detektorschicht bestimmte Weise deponiert, zum Beispiel in Form einer Lichtmenge, einer Ladungsmenge, einer Ionisation oder dergleichen.

Der Detektorschicht ist eine Auswerteeinrichtung zur unmittelbaren Bestimmung der in der Detektorschicht deponierten Strahlungsenergie der ionisierenden Strahlung zugeordnet. Durch diese Zuordnung zur Auswerteeinrichtung ist eine instantane bzw. zeitnahe Auswertung der Dosis in der Weise möglich, dass quantitative Dosisangaben für einen bestimmten Messort zu einer bestimmten Messzeit bzw. für ein bestimmtes, ggf. relativ kurzes Messzeitintervall verfügbar sind.

Die Detektorschichtdicke bestimmt das Messvolumen des Detektors nach Durchtritt der Strahlung durch das Absorbermaterial bzw. die Absorberschicht. Das mit Hilfe der Detektorschicht erzeugte Signal ist im Wesentlichen durch das Produkt aus Energie- oder Teilchenfluenz und Massen-Energieabsorptionskoeffizient µₑₙ/ρ bzw. Massen-Stoßbremsvermögen S/p bestimmt.

Wenn die Detektorschicht aus einem festen Detektorschichtmaterial besteht (Festkörperdetektor), kann die Detektorschicht wesentlich dünner sein als die Absorberschicht, um die Messtiefe möglichst genau festzulegen. Bei manchen Ausführungsformen von Dosismessgeräten mit Festkörperdetektor liegt die Detektorschichtdicke im Bereich von 0,01 mm bis 2 mm. Die Detektorschichtdicke kann vorzugsweise im Bereich von 1 mm oder weniger liegen, insbesondere im Bereich von ca. 50 µm bis 90 µm. Dadurch kann in der Regel ein guter Kompromiss zwischen linearem Integrationsbereich um 3 mm und ausreichendem Detektoransprechvermögen erzielt werden. Gleiche oder ähnlich Detektorschichtdicken sind auch flüssigen Detektorschichten bei Flüssigkeitsdetektoren günstig.

Es kann auch eine gasgefüllte Detektorschicht vorgesehen sein. Hier liegen brauchbare Detektorschichtdicken bei der beanspruchten Erfindung im Bereich von 1 mm bis 100 mm. Dies kann wie folgt verstanden werden. Bei einem Dichteskalierungfaktor von ca. 1000 zwischen Gas und gewebeäquivalentem Material würde man für einen möglichst punktförmigen Messpunkt von gewebeäqivalenten Materialdicken von 0,01 mm bis 2 mm auf etwa 10 mm bis 2000 mm Detektorschichtdicke für gasgefüllte Detektoren extrapolieren. Detektorschichtdicken im oberen Grenzbereich scheinen aber zu unhandlich und daher nicht erforderlich. Detektorschichtdicken bis ca. 100 mm sind in der Regel ausreichend. Dicken ab 1 mm für gasgefüllte Detektoren können bereits eine ausreichend gute Response geben.

Es hat sich herausgestellt, dass effektive Absorberschichtdicken, die deutlich kleiner sind als die angegebenen unteren Grenzen zum Beispiel für niederenergetische ß-Strahlung ein zu hohes Messsignal ergeben würden. Dadurch wäre keine zuverlässige dosisrichtige Messung der Augenlinsendosis möglich. Liegt dagegen die effektive Absorberschichtdicke deutlich oberhalb der oberen Grenzen, so kann in der Regel keine dosisrichtige Messung mehr erfolgen, beispielsweise weil Absorberschichtdicken von mehr als 10 mm keine oder fast keine Sr-90/Y-90-Elektronen mehr durchlassen.

Mit der vergleichsweise geringen Detektorschichtdicke soll erreicht werden, dass die deponierte Strahlungsenergie möglichst genau in derjenigen Tiefe (ausgehend von der Eintrittsseite der Absorberschicht) bestimmt wird, die bei der Definition der Dosis für die Augenlinse als zweckmäßig angesehen wurde.

Um eine noch präzisere Anpassung der Messergebnisse an die Definition der Augenlinsendosis zu erreichen, ist bei manchen Ausführungsformen vorgesehen, dass auf die Austrittsfläche der Detektorschicht in Durchstrahlungsrichtung ein Rückstreuelement aus einem Material folgt, welches einen Anteil von durch die Detektorschicht hindurchgelassener ionisierter Strahlung in Richtung der Detektorschicht zurückstreut. Das Rückstreuelement sollte vorzugsweise derart ausgelegt sein, dass es in Bezug auf die Wirkung auf eintretende ionisierende Strahlung einer Schicht aus gewebeäquivalentem Material mit einer Schichtdicke von 0,5 cm bis höchstens 30 cm, entsprechend dem gewünschten Energiebereich, entspricht. Zur Abschätzung können folgende Angaben hilfreich sein: Die Halbwertsschichtdicke (HWS) in ICRU-Gewebe für 2,3 MeV Bremsstrahlungsphotonen (von Sr-90/Y-90) Beta max. Energie 2,3 MeV) beträgt ca. 24 mm; bei der mittleren Energie von 0,93 MeV beträgt sie ca. 15mm. Bei der mittleren Energie für Pm-147 von 0,244 MeV ist die HWS ca. 8,5 mm. Für 50 keV Bremsstrahlungsphotonen mit einer HWS von 5 mm könnte hier eine untere Grenze festgelegt werden. Das ICRU-Weichteilgewebe ist ein für dosimetrische Zwecke definiertes gewebeäquivalentes Material der Dichte 1 g/cm3, das aus 76,2 % Sauerstoff, 11,1 % Kohlenstoff, 10,1 % Wasserstoff und 2,6 % Stickstoff besteht. Es können z.B. auch fast gleichwertiges Wasser oder Plastikmaterialien (z.B. Szintillatoren) verwendet werden.

Es gibt unterschiedliche Möglichkeiten der Ausgestaltung eines solchen Dosismessgeräts.

Bei einer Weiterbildung wird die Detektorschicht durch ein Szintillatormaterial gebildet und in Durchstrahlungsrichtung hinter der Detektorschicht ist ein optoelektronischer Wandler zur Umwandlung von Szintillationslicht in elektrische Signale angeordnet. Die Auswerteeinrichtung ist hierbei an den Wandler elektrisch angeschlossen. Vorzugsweise ist das Szintillatormaterial ein Plastikszintillatormaterial, welches gelöste organische Szintillatoren, gegebenenfalls zusammen mit anderen organischen Additiva, in einer Trägersubstanz in Form einer polymeren Festkörpermatrix aufweist. Im szintillierenden Medium des Szintillatormaterials wird Gamma-Strahlung oder Teilchenstrahlung in Szintillationslicht, beispielsweise sichtbares Licht, UV-Licht oder auch Röntgenstrahlung umgewandelt. Als optoelektronischer Wandler kann beispielsweise ein Photomultiplier verwendet werden, möglich sind aber z.B. auch Lawinenphotodioden (APDs), CCDs oder dergleichen.

Bei Ausführungsformen mit Rückstreuelement ist dann vorzugsweise zwischen der Detektorschicht und dem Wandler ein Rückstreuelement aus (mindestens) einem für das Szintillationslicht transparenten Material angeordnet. Falls das Rückstreuelement aus mehreren Materialien besteht muss mindestens eines in der Weise transparent sein, dass Licht zum Wandler gelangen kann. Das Material hat dann eine Doppelfunktion, indem es einerseits als Rückstreuelement wirkt und andererseits als Lichtleiter für das Szintillationslicht. Beispielsweise kann ein Lichtleiter aus Polymethylmetacrylat (PMMA) geeigneter Länge (beispielsweise zwischen ca. 0.5 cm und 10 cm) als Rückstreuelement/Lichtleiter genutzt werden. Für eine optimale Dimensionierung der Länge sind u.a. Reichweite und Rückstreuverhalten verschiedener Strahlungen zu berechnen (z.B. mittlere Betaenergie bei Sr-90/Y-90: 0,93 MeV - Reichweite in Gewebe 5 mm; Max. Betaenergie 2,3 MeV: Reichweite 13 mm. Die durch β-Teilchen induzierten Bremsstrahlungsphotonen gleicher Energien haben Halbwertsschichtdicken von 96 bzw. 154 mm)

Bei anderen Varianten mit Festkörperdetektor ist vorgesehen, dass die Detektorschicht im Wesentlichen aus einem Halbleitermaterial besteht und dass die Auswerteeinrichtung elektrisch an die Detektorschicht angeschlossen ist. Durch diese Anordnung wird direkt die in der Halbleiterschicht deponierte Ladungsmenge erfasst und ausgewertet. Diese korreliert mit der detektierten Strahlungsdosis. Das Halbleitermaterial kann beispielsweise im Wesentlichen aus Silizium, Germanium oder einem Verbindungshalbleiter, wie GaAs oder dergleichen, bestehen.

Bei einer anderen Weiterbildung weist die Detektorschicht eine flächig ausgedehnte Ionisationszähldrahtanordnung auf. Diese kann eine laterale Flächenausdehnung haben, welche größer ist als die Dicke in Durchstrahlungsrichtung. Es kann sich dabei um eine sehr flache, gasgefüllte Ionisationskammer mit einer Vielzahl zueinander paralleler Zähldrähte handeln, die ähnlich wie ein makroskopisches Zählrohr angeschlossen und ausgewertet werden kann.

Bei allen Varianten können Einrichtungen vorgesehen sein, die eine instantane Nutzung der Messergebnisse für einen Anwender erlauben. Bei manchen Ausführungsformen ist eine Messwertanzeige vorgesehen, um gemessene Dosiswerte anzuzeigen, z.B. in Form von Zahlen und/oder Diagrammen an einem Display. Die Messwerte können ggf. mit Daten für Ortsangaben bzw. mit Messpositionsdaten korreliert werden, um eine räumliche Verteilung von Dosiswerten anzeigen zu können.

Alternativ oder zusätzlich kann eine Sicherheitseinrichtung vorgesehen sein, die bei Überschreiten einer voreinstellbaren Alarmschwelle ein Alarm (z.B. akustisch und/oder optisch) ausgelöst. Dadurch wird der Nutzen des Dosismessgeräts im Sinne eines zuverlässigen Strahlenschutzes weiter erhöht.

### KURZBESCHREIBUNG DER ZEICHNUNGEN

Weitere Vorteile und Aspekte der Erfindung ergeben sich außer aus den Ansprüchen auch aus der nachfolgenden Beschreibung von bevorzugten Ausführungsbeispielen der Erfindung, die nachfolgend anhand der Figuren erläutert sind. Dabei zeigen:
Fig. 1 eine schematische Schnittansicht durch Komponenten eines Dosismessgeräts gemäß einem ersten Ausführungsbeispiel;
Fig. 2 eine schematische Schnittansicht durch Komponenten eines Dosismessgeräts gemäß einem zweiten Ausführungsbeispiel;
Fig. 3 eine schematische Schnittansicht durch Komponenten eines Dosismessgeräts gemäß einem dritten Ausführungsbeispiel;

### DETAILLIERTE BESCHREIBUNG DER AUSFÜHRUNGSBEISPIELE

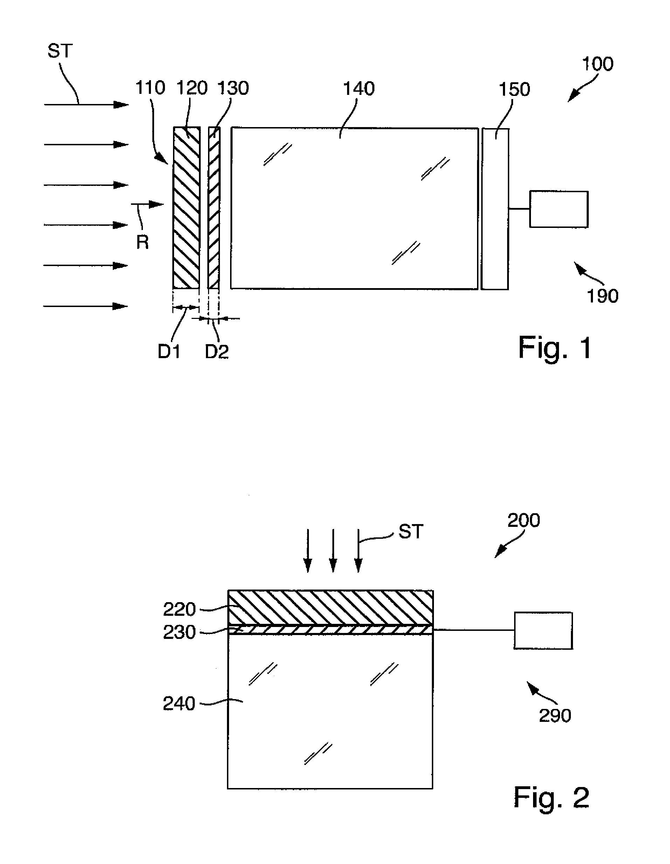

In Fig. 1 ist schematisch ein speziell für die Messung der Augenlinsendosis konzipiertes Dosismessgeräts 100 gemäß einem ersten Ausführungsbeispiel dargestellt. Das Dosismessgerät hat eine Strahlungseintrittsseite 110, auf die die durch Pfeile symbolisierte ionisierende Strahlung ST einfällt. Die Strahlung trifft zunächst auf eine Absorberschicht 120, die für die ionisierende Strahlung 110 teilweise absorbierend wirkt. Die Absorberschicht 120 ist im Beispiel als Teilbereich einer relativ biegesteifen, selbsttragenden Abdeckung aus einem Kunststoffmaterial ausgebildet. Das Absorberschichtmaterial ist ein gewebeäquivalentes Material, welches bezüglich seiner absorbierenden Wirkung auf durchtretende ionisierende Strahlung dem menschlichen Gewebe sehr ähnlich ist. Es kann sich beispielsweise um ein Kunststoffmaterial oder Plastikmaterial handeln oder um eine mit Wasser gefüllte dünne Kammer. Die in Durchstrahlungsrichtung R gemessene Absorberschichtdicke D1 ist über den gesamten genutzten Flächenbereich konstant, beträgt im Beispielsfall 3 mm und kann allgemein zum Beispiel zwischen 2,5 mm und 3,5 mm liegen. In der Absorberschicht wird ein Anteil der eintretenden ionisierenden Strahlung absorbiert, wobei die Intensität des aus der Austrittsfläche austretenden Strahlungsanteils durch die Absorberschichtdicke und das Absorberschichtmaterial bestimmt wird.

Unmittelbar anschließend trifft die durch die Absorberschicht hindurchgetretene ionisierende Strahlung auf eine Detektorschicht 130, die im Beispielsfall durch einen flachen, ebenen Plastikszintillator mit einer Schichtdicke zwischen 0,01 mm und 2 mm, insbesondere zwischen 50 µm und 90 µm, gebildet wird. Der Plastikszintillator dient als Strahlungsdetektor z.B. für Photonen und ß-Teilchen.

Die Detektorschicht bzw. der Plastikszintillator ist direkt auf eine Lichteintrittsseite eines Lichtleiters aus Plexiglas aufgebracht, der bei dieser Anordnung als Rückstreukörper 140 für diejenige Strahlung dient, die durch die Detektorschicht 130 hindurchgetreten ist. Über diesen Lichtleiter werden die wesentlichen Rückstreueffekte in der ICRU-Kugel physikalisch nachgebildet.

Das Szintillationslicht, welches durch den Plastikszintillator unter Einwirkung der eintretenden ionisierenden Strahlung erzeugt wird, gelangt zu einem Anteil über den Lichtleiter 140 auf die Eintrittsseite eines optoelektronischen Wandlers 150 in Form eines Photomultipliers. Der Photomultiplier ist ein Beispiel für einen Lichtdetektor für optische Photonen. Die Intensität der auftreffenden Strahlung wird anhand der vom Photomultiplier ausgegebenen elektrischen Signale nachgewiesen.

Im Beispielsfall werden die Analogimpulse des Photomultipliers über eine oder mehrere elektronische Schwellen diskriminiert. Wenn mehr als ein Zählsignal ausgewertet wird, erfolgt die Dosisberechnung über eine geeignete Linearkombination der Einzelzellraten. Die Schwellen werden dabei so gewählt, dass sich eine optimale Energieabhängigkeit ergibt. Der Photomultiplier und die daran angeschlossenen Komponenten (z.B. elektronische Bauteile zur Signalformung und Verstärkung, Datenaufnahmesystem, Computer) gehören zur Auswerteeinrichtung 190 des Dosismessgeräts.

Mit der Schichtdicke D2 des Plastikszintillators als Parameter kann das Verhältnis der Effizienz zwischen β-Teilchen und Photonenstrahlung optimiert werden. Die Detektorschichtdicke D2 liegt im Beispielsfall bei wenigen Zehntel Millimetern (z.B. bei ca. 0.1 mm), um einerseits ausreichend Szintillationslichtausbeute zu generieren und andererseits den Tiefenbereich, in welchem die Detektion stattfindet, möglichst präzise im Bereich von ca. 3 mm ausgehend von der Strahlungseintrittsfläche 110 des Detektors zu halten.

Das Dosismessgerät kann als tragbares Messgerät realisiert werden, bei dem die eigentliche Sonde mit der Kombination aus Absorberschicht und nachfolgender Detektorschicht an der Stirnseite eines Lichtleiters angebracht ist, der als Rückstreuelement fungiert und gleichzeitig Szintillationslicht zum Photodetektor der Auswerteeinrichtung leitet.

Der Aufbau des Dosismessgeräts ähnelt, mit der Ausnahme der Absorberschicht 120, dem Aufbau des in der EP 2 469 304 A2 beschriebenen Dosismessgeräts zur Messung der Richtungs-Äquivalentdosis. Der Detektor des dortigen Dosismessgeräts umfasst an der Lichteintrittsseite eines Plastikszintillators ein lichtundurchlässiges, flächiges, insbesondere ebenes Abdeckelement, welches hinsichtlich seiner Absorptionseigenschaften für durch das Abdeckelement tretende zu messende Strahlung im Wesentlichen identische Eigenschaften aufweist wie gewebeäquivalentes Material bzw. Gewebe mit einer Dicke von 0,07 mm. Bei einem zur Messung der Augenlinsendosis optimierten Dosismessgerät ist die entsprechende Absorberschicht dagegen wesentlich dicker, da vorzugsweise eine Absorberschichtdicke von ca. 3 mm vorgesehen wird, wenn gewebeäquivalentes Material verwendet wird.

Ein Dosismessgerät der in EP 2 469 304 A2 gezeigten Art kann in ein Dosismessgerät zur Messung der Augenlinsendosis umgerüstet werden, indem zusätzlich zu der ca. 0,07 mm dicken Abdeckung noch eine weitere Abdeckung aufgebracht wird, deren Wirkung auf ionisierende Strahlung zum Beispiel einer Absorberschichtdicke von 2,93 mm (± 20%) entspricht, so dass die bereits vorgesehene, nur 70 µm dicke Abdeckung inklusive der weiteren Abdeckung im Wesentlichen die gleiche absorbierende Wirkung hat wie eine Absorberschicht gemäß der hier beanspruchten Erfindung.

Alternativ könnte das dort beschriebene dünne Abdeckelement durch eine dickere Absorberschicht der hier beschriebenen Art ersetzt werden. Durch entsprechende Modifikationen des in der EP 2 469 304 A2 beschriebenen Dosismessgeräts wäre also eine dosisrichtige Messung der Augenlinsendosis möglich. Daher wird hier auch die Verwendung des dort beschriebenen Dosismessgeräts mit der entsprechenden Modifikation zur Messung der Augenlinsendosis vorgeschlagen.

Das Messgerät kann als Mehrzweck-Dosismessgerät ausgelegt werden, indem auswechselbare Absorberelemente (z.B. in Form geeigneter Abdeckelemente) bereitgestellt werden, die zum Beispiel für die Messung der Hautdosis einer Absorberschichtdicke von ca. 0,07 mm eines gewebeäquivalenten Materials entsprechen und im anderen Fall, für die Messung der Augenlinsendosis, einer Absorberschichtdicke mit der Wirkung einer ca. 3 mm (d.h. 2 mm bis 4 mm) dicken Absorberschicht aus gewebeäquivalentem Material.

Als Alternative zum Plastikszintillator kann auch ein Flüssigszintillator gleicher oder ähnlicher Dicke verwendet werden, um anstelle eines Festkörperdetektors einen Flüssigkeitsdetektor zu bilden. Bei dem flüssigen Szintillatormaterial kann es sich z.B. um Wasser, einen flüssigen Kohlenwasserstoff (z.B. Toluol, Benzol) oder eine ähnliche Flüssigkeit handeln, die mit Szintillatormolekülen versetzt ist.

Fig. 2 zeigt ein zweites Ausführungsbeispiel eines Dosismessgeräts 200 zur Messung der Augenlinsendosis. Hier ist eine ca. 3 mm dicke Absorberschicht 220 direkt auf eine ca. 100 µm dicke Detektorschicht 130 aus einem Halbleitermaterial aufgebracht. Auch geringere Dicken der Detektorschicht sind möglich, aber ggf. schwerer mit der erforderlichen Qualität herstellbar. Die dünne Detektorschicht ist mittels einer geeigneten Dünnschichttechnologie direkt auf eine Lichteintrittsseite eines Rückstreuelements 240 in Form eines Plexiglaskörpers mit einer Dicke von ca. 5 mm bis 10 mm aufgebracht. Die Halbleiterschicht (Detektorschicht 230) ist über elektrische Anschlussleitungen mit einer Auswerteeinrichtung 290 verbunden. Derjenige Strahlungsanteil, der durch die Absorberschicht 220 hindurch in die Detektorschicht 230 eintritt, sowie diejenigen Strahlungsanteile, die nach Durchtritt durch die Absorberschicht und die Detektorschicht in das Rückstreuelement 240 eintreten und in Richtung Detektorschicht zurückgestreut werden, führen in der Halbleiterschicht zur Erzeugung elektrischer Ladungen, deren Ladungsmengen proportional zur aufgenommen Strahlungsdosis ist. Die Auswerteeinrichtung 290 ist zur quantitativen Auswertung dieser elektrischen Ladungsmenge geeignet. Sie kann z.B. einen Vorverstärker, einen Verstärker, einen Diskriminator oder Einkanal- oder Vielkanalanalysator, eine Prozessoreinheit zur Verarbeitung der Signale und ähnliche Komponenten enthalten.

Anstelle einer ca. 3 mm dicken Kunststoff-Absorberschicht könnten auch andere Absorberschichten genutzt werden, die im Wesentlichen die gleiche absorbierende Wirkung auf ionisierende Strahlung haben. Beispielsweise könnte die Absorberschicht durch eine dünne Folie aus Titan oder einem anderen Metall mittlerer Ordnungszahl oder eine mit einer Metallschicht geeigneter Dicke beschichtete Kunststofffolie gebildet sein.

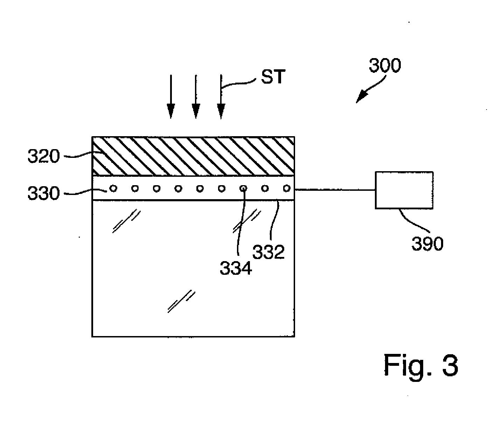

In Fig. 3 ist ein drittes Ausführungsbeispiel eines Dosismessgeräts 300 zur Messung der Augenlinsendosis gezeigt. Zur Strahlungsdetektion ist ein flächig ausgedehnter gasgefüllter Proportionalzähler oder eine flache Ionisationskammer 330 vorgesehen, der bzw. die als Detektorschicht fungiert und zwischen einer eintrittsseitigen Absorberschicht 320 (gewebeäquivalente Kunststoffschicht mit ca. 3 mm Schichtdicke) und einem austrittsseitigen Rückstreuelement 340 eingefügt ist. Die Anordnung mit einem gasgefüllten Strahlungsdetektor hat ein senkrecht zur Durchstrahlungsrichtung flächig ausgedehntes, in Durchstrahlungsrichtung nur ca. 1 mm bis 100 mm dickes Gehäuse 332, das eine mit Zählgas gefüllte Kammer bildet. Innerhalb der Kammer (Detektorkammer) verlaufen zahlreiche zueinander parallele, elektrisch leitende Zähldrähte 334, die jeweils an die Auswerteeinrichtung 390 elektrisch angeschlossen sind. Die Funktion der Anordnung mit einem gasgefüllten Detektor entspricht im Wesentlichen der Funktion bekannter Zählrohre oder Ionisationskammern, weshalb auf eine detaillierte Beschreibung hier verzichtet wird.

Allen Ausführungsbeispielen ist gemeinsam, dass sie aufgrund ihrer Konstruktion und der Dimensionierung der für die Strahlungserfassung wichtigen Komponenten (Absorberschicht, Detektorschicht, gegebenenfalls Rückstreuelement) eine direkte, dosisrichtige Messung der Augenlinsendosis mit hoher örtlicher und zeitlicher Auflösung ermöglichen. Damit können beispielsweise inhomogene und/oder größere Strahlungsfelder im Hinblick auf die Existenz von besonders strahlenbelasteten Bereichen und/oder weitgehend unbelasteten Bereichen analysiert werden.

## Patentansprüche

1. Dosismessgerät zur Messung der Augenlinsendosis umfassend:
eine für eintretende ionisierende Strahlung teilweise absorbierende Absorberschicht (120, 220, 320), die an einer Strahlungseintrittsseite (110) des Dosismessgeräts (100, 200, 300, 400) angeordnet ist und eine zwischen einer Eintrittsfläche und einer Austrittsfläche der Absorberschicht gemessene effektive Absorberschichtdicke (D1) aufweist;
eine auf die Austrittsfläche der Absorberschicht in Durchstrahlungsrichtung folgende Detektorschicht (130, 230, 330), die eine zwischen einer Eintrittsfläche und einer Austrittsfläche der Detektorschicht gemessene wirksame Detektorschichtdicke (D2) aufweist, und
eine der Detektorschicht zugeordnete Auswerteeinrichtung (190, 290, 390, 490) zur Bestimmung der in der Detektorschicht deponierten Strahlungsenergie der ionisierenden Strahlung,
**dadurch gekennzeichnet, dass**
die effektive Absorberschichtdicke (D1) an ein Absorberschichtmaterial oder eine Kombination mehrerer Absorberschichtmaterialien so angepasst ist, dass die Absorberschicht in Bezug auf die Wirkung auf hindurchtretende ionisierende Strahlung einer Schicht aus gewebeäquivalentem Material mit einer Schichtdicke zwischen 2 mm und 4 mm entspricht; und
die Detektorschichtdicke (D2) bei einem Festkörperdetektor oder Flüssigkeitsdetektor im Bereich von 0.01 mm bis 2 mm und bei einem gasgefüllten Detektor im Bereich von 1 mm bis 100 mm liegt; und
die Auswerteeinrichtung zur unmittelbaren Bestimmung der in der Detektorschicht deponierten Strahlungsenergie der ionisierenden Strahlung konfiguriert ist.

2. Dosismessgerät nach Anspruch 1, **dadurch gekennzeichnet, dass** die effektive Absorberschichtdicke (D1) so bemessen ist, dass ihre Absorptionswirkung einer Schicht aus gewebeäquivalentem Material mit einer Schichtdicke zwischen 2,5 mm und 3,5 mm, insbesondere zwischen 2,8 mm und 3,2 mm, entspricht.

3. Dosismessgerät nach Anspruch 1 oder 2, **dadurch gekennzeichnet, dass** die Detektorschichtdicke (D2) bei dem Festkörperdetektor oder Flüssigkeitsdetektor im Bereich von 1 mm oder weniger, insbesondere im Bereich von ca. 50 µm bis 90 µm liegt.

4. Dosismessgerät nach einem der vorhergehenden Ansprüche, **gekennzeichnet durch** ein auf die Austrittsfläche der Detektorschicht in Durchstrahlungsrichtung folgendes Rückstreuelement (140, 240, 340) aus einen Material, das einen Anteil von **durch** die Detektorschicht hindurchgelassener ionisierender Strahlung in Richtung der Detektorschicht zurückstreut, wobei das Rückstreuelement vorzugsweise derart ausgelegt ist, dass es in Bezug auf die Wirkung auf eintretende ionisierende Strahlung einer Schicht aus gewebeäquivalentem Material mit einer Schichtdicke von 0,5 cm bis höchstens 30 cm entspricht.

5. Dosismessgerät nach einem der vorhergehenden Ansprüche, **dadurch gekennzeichnet dass** die Detektorschicht (130) durch einen Szintillatormaterial gebildet ist, insbesondere durch ein Plastikszintillatormaterial, und dass in Durchstrahlungsrichtung (R) hinter der Detektorschicht ein optoelektronischer Wandler (150), insbesondere ein Photomultiplier, zur Umwandlung von Szintillationslicht in elektrische Signale angeordnet ist.

6. Dosismessgerät nach Anspruch 5, **dadurch gekennzeichnet dass** zwischen der Detektorschicht (130) und dem optoelektronischen Wandler (150) ein Rückstreuelement (140) aus mindestens einem für Szintillationslicht transparentem Material angeordnet ist, insbesondere aus Polymethylmethacrylat.

7. Dosismessgerät nach einem der Ansprüche 1 bis 4, **dadurch gekennzeichnet dass** die Detektorschicht (230) im Wesentlichen aus einem Halbleitermaterial besteht und dass die Auswerteeinrichtung (290) elektrisch an die Detektorschicht angeschlossen ist.

8. Dosismessgerät nach einem der Ansprüche 1 bis 4, **dadurch gekennzeichnet dass** die Detektorschicht (330) eine flächig ausgedehnte Anordnung mit einem gasgefüllten Strahlungsdetektor aufweist.

9. Dosismessgerät nach einem der vorhergehenden Ansprüche, **dadurch gekennzeichnet, dass** es als Mehrzweck-Dosismessgerät ausgelegt ist, wobei dem Dosismessgerät auswechselbare Absorberelemente zugeordnet sind, die für die Messung der Hautdosis einer Absorberschichtdicke von 0,07 mm eines gewebeäquivalenten Materials und für die Messung der Augenlinsendosis einer Absorberschichtdicke zwischen 2 mm und 4 mm eines gewebeäquivalenten Materials entsprechen.

10. Dosismessgerät nach einem der vorhergehenden Ansprüche, **gekennzeichnet durch** eine Messwertanzeige zur Anzeige von Dosismesswerten.

11. Dosismessgerät nach einem der vorhergehenden Ansprüche, **gekennzeichnet durch** eine Sicherheitseinrichtung, die konfiguriert ist, bei Überschreiten einer voreinstellbaren Alarmschwelle ein Alarm, insbesondere einen optischen und/oder einen akustischen Alarm, auszulösen.
